(19) Europäisches Patentamt
European Patent Office
Office européen des brevets

(11) Veröffentlichungsnummer : **0 078 005**
**B1**

(12) # EUROPÄISCHE PATENTSCHRIFT

(45) Veröffentlichungstag der Patentschrift : **07.08.85**

(51) Int. Cl.⁴ : **C 07 C118/00**

(21) Anmeldenummer : **82109669.0**

(22) Anmeldetag : **20.10.82**

(54) **Verfahren zur Herstellung von Isocyanaten durch thermische Spaltung von Urethanen.**

(30) Priorität : **28.10.81 DE 3142627**

(43) Veröffentlichungstag der Anmeldung : **04.05.83 Patentblatt 83/18**

(45) Bekanntmachung des Hinweises auf die Patenterteilung : **07.08.85 Patentblatt 85/32**

(84) Benannte Vertragsstaaten : **AT BE CH DE FR GB IT LI NL SE**

(56) Entgegenhaltungen :
**DE-A- 2 410 505**
**FR-A- 2 320 288**

(73) Patentinhaber : **BASF Aktiengesellschaft**
**Carl-Bosch-Strasse 38**
**D-6700 Ludwigshafen (DE)**

(72) Erfinder : **Merger, Franz, Dr.**
**Max-Slevogt-Strasse 25**
**D-6710 Frankenthal (DE)**
Erfinder : **Nestler, Gerhard, Dr.**
**Van-Leyden-Strasse 17**
**D-6700 Ludwigshafen (DE)**
Erfinder : **Platz, Rolf, Dr.**
**Hansastrasse 5**
**D-6800 Mannheim (DE)**
Erfinder : **Towae, Friedrich, Dr.**
**Parkstrasse 22**
**D-6700 Ludwigshafen (DE)**
Erfinder : **Hellbach, Hans**
**Stettiner Strasse 14**
**D-6840 Lampertheim (DE)**

**EP 0 078 005 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, daß N-substituierte Urethane thermisch in der Gasphase oder in flüssiger Phase in Isocyanate spaltbar sind. Bei der thermischen Spaltung treten vielfach zusätzlich verschiedene unerwünschte Nebenreaktionen auf. Genannt seien beispielsweise die Decarboxylierungsreaktion der Urethane, die von der Bildung primärer und sekundärer Amine sowie von Olefinen begleitet sein kann; Umsetzungen zwischen dem gebildeten Isocyanat und Urethan zu Allophanaten bzw. Amin zu Harnstoffen und Polymerisation der Isocyanate zu Isocyanuraten.

Die Pyrolyse der Urethane in der Dampfphase wird nach Angaben der DE-OS 19 44 719 (GB-PS 1 247 451) bei Temperaturen von 400 bis 600 °C in Gegenwart von Lewissäure als Katalysator durchgeführt, wobei das Isocyanat und der Alkohol durch fraktionierte Kondensation getrennt werden. Toluylen-diisocyanat wird z. B. durch Pyrolyse von Toluylen-2,4-diethylurethan in Gegenwart von Eisen-(III)-chlorid erhalten. Nachteile der Reaktion sind u. a. niedrige Ausbeuten, verbunden mit beträchtlichen Mengen eines polymeren Nebenproduktes, Zersetzung des Katalysators und Korrosion der Reaktionsapparatur. In der DE-OS 24 10 505 (US 3 870 739) wird ein Verfahren beschrieben, bei dem das Urethan bei einer Temperatur von 350 bis 550 °C und einem Druck von weniger als dem (m + 1) fachen des Isocyanatdampfdruckes in einer katalysatorfreien Pyrolysezone innerhalb von 15 Sekunden gespalten wird. Nachteilig an diesem Verfahren ist u. a., daß eine große, für die endotherme Spaltung erforderliche Wärmemenge innerhalb einer sehr kurzen Zeit dem pulverförmigen Urethan zugeführt werden muß und ein als Nebenprodukt anfallendes festes Polymer und dessen Abtrennung die Durchführung eines kontinuierlichen Verfahrens erschwert.

Die thermische Spaltung von Urethanen in flüssiger Phase wird beispielsweise in den DE-AS 24 21 503 (US 3 962 302) und DE-AS 25 30 001 (US 3 919 280) beschrieben. Nach Angaben der DE-AS 24 21 503 werden die Urethane in einem inerten Lösungsmittel, wie Alkylbenzolen, linearen und cyclischen Kohlenwasserstoffen und/oder Phthalsäureestern, gelöst und unter Normal- oder Überdruck bei 175 °C bis 350 °C gespalten. Die Isolierung und Trennung des erhaltenen Isocyanates und Alkohols erfolgt mit Hilfe des Lösungsmittels als Trägermittel und/oder unter Verwendung eines Inertgases als Trägermittel. Gemäß DE-AS 25 30 001 werden als Reaktionsmedium höhermolekulare, gegebenenfalls substituierte aliphatische, cycloaliphatische oder aromatische Kohlenwasserstoffe, Ether, Ester oder Ketone verwendet. Zur Trennung der Spaltprodukte wird die Destillation genannt, wobei über Kopf Isocyanat, Alkohol und Trägermaterial abdestilliert werden, während das Reaktionsmedium als Bodenfraktion zurückbleibt.

Zur Herstellung von aromatischen Isocyanaten werden die Urethane nach DE-OS 26 35 490 (US 4 081 472) bei Temperaturen von 150 bis 350 °C unter vermindertem Druck mit einer Lösung aus wenigstens einem Metallion, wie Ionen von Kupfer, Zink, Aluminium, Zinn, Titan, Vanadium, Eisen, Cobalt und Nickel als Katalysator, der in einem Lösungsmittel mit einem Siedepunkt von 200 °C in einer Metallkonzentration von wenigstens 0,001 Gew. %, bezogen auf das Lösungsmittel, gelöst ist, in Kontakt gebracht. Die Trennung der erhaltenen Spaltprodukte erfolgt durch fraktionierte Kondensation. Hierbei bleiben jedoch in geringen Mengen gebildete, nicht destillierbare Polymerisationsprodukte im Katalysator enthaltenden Lösungsmittelrückstand, wodurch nach einiger Zeit zusätzlich Reinigungsoperationen der die katalytisch wirksamen Metallionen enthaltenden Lösungsmittel notwendig werden.

Nach EP-OS 28 724 erzielt man sehr gute Spaltergebnisse, wenn man Urethane an katalytisch wirksamen, oberflächenreichen Metallen, die in heterogener Phase vorliegen, spaltet. Nachteilig an diesem Verfahren ist, daß die als Katalysatoren eingesetzten Metalle mit der Zeit durch Belegung ihre katalytische Aktivität verlieren, wodurch ebenfalls zusätzliche Reinigungsoperationen notwendig werden.

Aufgabe der vorliegenden Erfindung war es, ein Verfahren zur thermischen Spaltung von Urethanen in die entsprechenden Isocyanate zu entwickeln, bei dem die bei den bekannten Verfahren bestehenden Mängel beseitigt oder zumindest verringert werden.

Überraschenderweise wurde gefunden, daß man Urethane auf einfache Weise und mit sehr guten Ausbeuten in Gegenwart von Kohlenstoff thermisch spalten kann.

Gegenstand der Erfindung ist somit ein Verfahren zur Herstellung von Isocyanaten durch thermische Spaltung von Urethanen bei Temperaturen von 175 bis 600 °C, das dadurch gekennzeichnet ist, daß man die Spaltung in Gegenwart von Kohlenstoff durchführt.

Der erfindungsgemäß verwendbare Kohlenstoff ist nicht nur ein guter Wärmeüberträger, sondern auch ein preisgünstiges Material, das nach längerem Einsatz, wenn es durch polymere Neben- oder Zersetzungsprodukte beispielsweise desaktiviert oder verklumpt ist, nicht regeneriert zu werden braucht, sondern durch Verbrennen rückstandsfrei und umweltfreundlich vernichtet werden kann.

Der Kohlenstoff besitzt zweckmäßigerweise ein Schüttgewicht von 700 bis 1 000 g/l, vorzugsweise von 750 bis 950 g/l. Er kann in Form von Pulver oder Preßlingen eingesetzt werden. Pulverförmiger Kohlenstoff weist eine durchschnittliche Korngröße von 0,01 bis 10 mm, vorzugsweise von 0,1 bis 2 mm auf. Die Preßlinge können beispielsweise die Form von Kugeln, Ringen, Zylindern oder Tabletten haben. Bei Verwendung von kugelförmigen Kohlenstoff-Preßlingen besitzen die Kugeln im allgemeinen Durchmesser von 0,2 bis 10 mm, vorzugsweise 0,5 bis 5 mm. Als zylinderförmige Preßlinge werden im allgemeinen Zylinder mit einer Länge von 2 bis 15 mm und einem Durchmesser von 2 bis 6 mm verwendet.

Nicht kugel- oder zylinderförmige Preßlinge weisen im allgemeinen ein Volumen auf, das dem von zylinderförmigen Preßlingen entspricht.

Der pulverförmige Kohlenstoff oder die Kohlenstoffpreßlinge können in einem Reaktionsgefäß, beispielsweise einem Röhren- oder Schachtofen in Form eines Festbettes aufgeschüttet werden. Die gewählte Dimension des Kohlenstoffestbettes wird vorzugsweise von der Möglichkeit bestimmt, die Spaltwärme zu übertragen.

Die Reaktionsgefäße, die im allgemeinen säulenförmig sind, können einen Querschnitt in beliebiger Form, beispielsweise in Form eines Quadrates oder einer Ellipse, aufweisen. Vorzugsweise verwendet man langgestreckte, zylinderförmige Reaktionsgefäße. Das Verhältnis von Innendurchmesser zu Länge des Reaktionsgefäßes beträgt im allgemeinen 1 : 2 bis 1 : 100, vorzugsweise 1 : 10 bis 1 : 40. Die Reaktionsgefäße können senkrecht oder waagrecht ausgerichtet sein und auch Zwischenlagen einnehmen. Vorzugsweise verwendet werden senkrecht stehende Röhrenofen, bei denen der Rohrdurchmesser etwa 10 bis 100 mm beträgt.

Die Spaltung der Urethane nach dem erfindungsgemäßen Verfahren wird jedoch vorzugsweise in einem gerührten Kohlenstoffbett oder in einem Wirbelbett mit Kohlenstoffschüttung durchgeführt.

Als Reaktionsgefäß eignet sich beispielsweise ein mit Kohlenstoff gefüllter und mit einem Wendel- oder Ankerrührer ausgestatteter Behälter. Zweckmäßig wählt man Behälter mit einem Verhältnis von Höhe zu Durchmesser von 0,5 bis 2,5 : 1, vorzugsweise von 0,7 bis 2 : 1 und einem Verhältnis von Höhe des Rührers im Gefäß (gemessen als vertikaler Abstand der Eintrittsöffnung des Rührers zum Niveau der Rührerspitze) zur Höhe des Kohlenstoffbettes von 0,8 bis 1 : 1.

Zur Durchführung des erfindungsgemäßen Verfahrens eignen sich ferner übliche Wirbelbettanlagen, die als Feststoffschüttung Kohlenstoff enthalten. Vorzugsweise verwendet werden jedoch Sprühwirbelbettanlagen, die im wesentlichen bestehen aus einem vorzugsweise zylinderförmigen Wirbelbehälter, dessen Innendurchmesser zur Länge im allgemeinen 1 : 0,5 bis 1 : 7, vorzugsweise 1 : 1 bis 1 : 3 beträgt, mit dem Wirbelbett, dem Anströmboden für das Wirbelgas, der beispielsweise aus einer Frittenplatte, einem Rundloch oder Glockenboden oder einem Conidur-Feinlochblech bestehen kann, einer Düse für die Produktzufuhr und der Produkt- und Abgasabfuhr, die gegebenenfalls über ein im Wirbelbehälter befindliches Filter oder über Zyklone erfolgen kann, sowie gegebenenfalls einer mechanischen Zerkleinerungsvorrichtung für die Kohle mit eventuell vorgeschalteter Klassiereinrichtung, beispielsweise einer Mühle oder einem rotierenden Schlagkreuz, die innerhalb oder außerhalb des Wirbelbehälters angeordnet sein kann und einer eventuell auftretenden Kornvergrößerung entgegenwirkt sowie neue Granulationskeime schafft, und Heizflächeneinbauten.

Überraschend war hierbei insbesondere, daß die Urethane, die dampfförmig, gelöst oder in geschmolzener Form in die Wirbelschicht eingedüst werden, nicht die Öffnungen im Anströmboden des Wirbelreaktors verstopfen oder gemeinsam mit den Spaltprodukten zu größeren, unter wirtschaftlichen Bedingungen nicht mehr wirbelfähigen Agglomeraten zusammenbacken. Trotz der breiten Verweilzeitverteilung im Wirbelbett werden die gebildeten Isocyanate thermisch praktisch nicht geschädigt.

Vorteilhaft ist ferner, daß die Wirbelbettanlage keine mechanisch bewegten Teile besitzt. Daraus ergeben sich eine geringe Reparaturanfälligkeit, geringe Wartungs- und Investitionskosten und hohe Raum-Zeit-Ausbeuten. Da die Abtrennung der Spaltprodukte üblicherweise bei Normaldruck erfolgt, treten keine Dichtungsprobleme auf. Die erforderliche Wärme kann einfach über das Wirbelgas zugeführt und an das Wirbelbett übertragen oder über im Wirbelbett befindliche Einbauten übertragen werden.

Schließlich ist ein weiterer Vorteil des erfindungsgemäßen Verfahrens, daß der verbrauchte Kohlenstoffrückstand in einer leicht handhabbaren, rieselfähigen und staubarmen Form anfällt.

Nach dem erfindungsgemäßen Verfahren wird das Kohlenstoffbett zur thermischen Spaltung der Urethane mit 20 bis 1 000, vorzugsweise 50 bis 500 Urethanäquivalenten/Liter Kohlenstoff und Stunde beaufschlagt.

Die thermische Spaltung kann bei Temperaturen von 175 bis 600 °C, vorzugsweise 220 bis 450 °C, diskontinuierlich oder kontinuierlich bei vermindertem, normalem oder erhöhtem Druck, beispielsweise bei Drücken von 0,01 bis 5 bar, vorzugsweise 0,1 bis 1,0 bar, durchgeführt werden.

Die Urethane können dem Kohlenstoffbett — wie bereits erwähnt — dampfförmig, in flüssiger oder fester Form, z. B. als Pulver, als Suspension oder vorzugsweise als Lösung in einem unter den Reaktionsbedingungen inerten Lösungsmittel zugeführt werden.

Die Spaltung der Urethane und Trennung der Spaltprodukte durch Abdestillieren des Alkohols, des Isocyanates und gegebenenfalls des Lösungsmittels können gleichzeitig oder nacheinander erfolgen. Um eine lange Standzeit des Kohlenstoffbetts und optimale Isocyanatausbeuten zu erreichen, werden die Spaltprodukte mit einem Strippmittel möglichst quantitativ aus dem Kohlenstoffbett ausgestrippt. Als Strippmittel eignen sich die Lösungsmittel, wobei solche Strippmittel vorzugsweise Verwendung finden, deren Siedepunkt zwischen den Siedepunkten des entstehenden Isocyanats und Alkohols liegt.

Zweckmäßigerweise wird man die Urethane in einem Lösungsmittel, das gleichzeitig als Strippmittel geeignet ist, gelöst in das Kohlenstoffbett einbringen, wobei Konzentrationen an Urethan im Lösungsmittel von 1 bis 70 Gew. %, vorzugsweise 5 bis 50 Gew. % vorteilhaft sind. Es ist jedoch auch möglich, das Strippmittel gesondert dem Kohlenstoffbett zuzuführen. Wird kein Lösungsmittel zur Spaltung verwendet, so muß ein Strippmittel zusätzlich in das Kohlenstoffbett eingeleitet werden, wobei das Gewichtsverhältnis von Urethan zu Strippmittel 1 : 0,5 bis 20, vorzugsweise 1 : 1 bis 10 beträgt. Weiterhin

ist es möglich, zusätzlich zu dem Strippmittel ein Inertgas, z. B. Kohlenmonoxid oder vorzugsweise Stickstoff, durch das Kohlenstoffbett zu leiten, sofern nicht schon ein Inertgas zum Verwirbeln des Kohlenstoffbetts verwendet wird. Die gebildeten Spaltprodukte und das Lösungs- und/oder Strippmittel leitet man in einer bevorzugten Ausführungsform in eine oder mehrere Trennkolonnen, wobei gegebenenfalls unter Benutzung des Strippmittels als Zwischensieder Alkohol und Isocyanat getrennt werden. Auch ist es möglich, die Spaltprodukte, Lösungs- und Strippmittel fraktioniert zu kondensieren.

Als Lösungs- oder Strippmittel seien beispielhaft genannt : Aliphatische Kohlenwasserstoffe, wie die höheren Alkane Decan, Undecan, Dodecan, Tridecan, Tetradecan, Pentadecan, Hexadecan, Heptadecan, Dekalin, flüssiges Paraffin, Erdölfraktionen von Paraffinen, die üblicherweise als Schmieröle, Kühlöle oder Schneidöle verwendet werden ; alicyclische Kohlenwasserstoffe, wie Erdölfraktionen der Naphthenreihe ; gegebenenfalls substituierte aromatische Kohlenwasserstoffe, wie z. B. Naphthalin, 1- und 2-Methylnaphthalin, 1,2-, 1,4-, 1,6-, 2,7-, 2,6- und 2,3-Dimethylnaphthalin, 1-Ethylnaphthalin, Phenylnaphthalin, Benzylnaphthalin, Toluol, 1,2-, 1,3- und 1,4-Dimethylbenzol, 1,2,4- und 1,3,5-Trimethylbenzol, 1,2,3,5- und 1,2,4,5-Tetramethylbenzol, 1,3,5-Triethylbenzol, Hexyl-, Heptyl-, benzol, Hexaethylbenzol, Diphenyl, 4,4'-Dimethyldiphenyl, Dibenzyl, Diphenylmethan und 4,4'-Dimethyl-diphenylmethan, halogensubstituierte aromatische Kohlenwasserstoffe, wie z. B. Chlorbenzol, 1,2- und 1,4-Dichlorbenzol, 1,4-Dijodbenzol, 1,2,3- und 1,3,5-Trichlorbenzol, 1,2,3,4-, 1,2,3,5- und 1,2,4,5-Tetrachlorbenzol, Pentachlorbenzol, 1- und 2-Fluornaphthalin, 1- und 2-Chlornaphthalin, 1- und 2-Jodnaphthalin und Diphenyldichlormethan ; Nitrogruppen haltige aromatische Kohlenwasserstoffe, wie z. B. Nitrobenzol, 3-Nitrotoluol, 2-Nitro-m-xylol, 5-Nitro-m-xylol und 4-Nitroanisol, aliphatische und aromatische Ketone, wie z. B. Cyclohexanon, Cycloheptanon, Di-n-butylketon, Di-n-amylketon, α-Tetralon, Acetophenon, Propiophenon, Benzophenon, 3-Methylbenzophenon, Dodecanon-2 und Tridecanon-2, Sulfone und Carbonsäureester, wie z. B. Sulfolan, Diethylsulfon, Phthalsäuredimethylester, Phthalsäurediethylester, Benzoesäurepropylester und Laurinsäureethylester und Ether, wie z. B. Diethylenglykol-dimethylether, Diethylenglykoldiethylether, Diisoamylether, Di-n-amylether, Resorcindimethylether, Resorcindiethylether, Phenyloctylether, Phenylbenzylether, Dibenzylether, Diphenylether, α-Methylnaphthylether und β-Ethylnaphthylether.

Besonders bewährt haben sich und daher vorzugsweise verwendet werden gegebenenfalls substituierte aromatische Kohlenwasserstoffe wie Benzol, Toluol, 1,2-, 1,3- und 1,4-Dimethylbenzol, 1,2,4- und 1,3,5-Trimethylbenzol, 1,2,3,5- und 1,2,4,5-Tetramethylbenzol, 1,3,5-Triethylbenzol, Hexyl-, Heptyl-, Octyl-, Nonyl-, Decylbenzol, halogensubstituierte aromatische Kohlenwasserstoffe, wie z. B. Nitrobenzol, 3-Nitrotoluol, 2-Nitro-m-xylol, 5-Nitro-m-xylol und 4-Nitroanisol, aliphatische und aromatische Ketone, wie z. B. Cyclohexanon, Cycloheptanon, Di-n-butylketon, Di-n-amylketon, Ether, wie z. B. Diethylenglykoldimethylether, Diethylenglykoldiethylether, Diisoamylether, Di-n-amylether.

Urethane, die nach dem erfindungsgemäßen Verfahren thermisch in Isocyanat und Alkohol gespalten werden, besitzen die allgemeine Formel

$$R(NHCOOR')_n,$$

in der bedeuten :

R einen heterocyclischen Rest oder vorzugsweise einen gegebenenfalls substituierten Aryl- und/oder Alkylrest, wobei der Alkylrest linear, verzweigt oder cyclisch sein kann und gegebenenfalls noch Heteroatome wie z. B. Schwefel, Sauerstoff oder Stickstoff und der Arylrest Methylengruppen als Brückenglieder gebunden enthalten kann,

R' einen gegebenenfalls substituierten aliphatischen, aromatischen oder aromatisch-aliphatischen Rest mit 1 bis 20 Kohlenstoffatomen, vorzugsweise 1 bis 10 Kohlenstoffatomen oder einen cycloaliphatischen Rest mit 3 bis 15 Kohlenstoffatomen, vorzugsweise 3 bis 7 Kohlenstoffatomen und

n eine ganze Zahl von 1 bis 4 und höher, vorzugsweise 1 bis 3 und insbesondere 2.

Als Reste R seien im Falle der gegebenenfalls substituierten Arylurethane beispielsweise genannt die Reste von aromatischen Monoaminen, wie Anilin, substituierten Anilinen, wie in 2-, 3- und/oder 4-Stellung durch eine Nitro-, Methyl-, Ethyl-, n-Propyl-, Isopropyl-, n-Butyl-, Isobutyl-, sek.-Butyl-, tert.-Butylgruppe oder ein Chloratom substituierten Anilinen, ortho-, meta- und/oder para-Hydroxy-, Methoxy-, Ethoxy-, Propoxy-, Isopropoxy-, N-Butoxy-, Isobutoxy-, sek.-Butoxy- und tert.-Butoxyanilin ; durch eine Aminogruppe in m- und/oder p-Stellung substituierte Benzoesäurealkylester mit 1 bis 4 Kohlenstoffatomen im Alkylrest, durch eine Aminogruppe in m- und/oder p-Stellung substituierte N-Alkoxycarbonylaminobenzole und -toluole mit 1 bis 4 Kohlenstoffatomen im Alkylrest ; α- und β-Naphthylamin ; aromatischen Diaminen wie 1,3- und 1,4-Diaminobenzol ; durch eine Nitro-, Methyl-, Ethyl-, n-Propyl-, Isopropyl-, n-Butyl-, Isobutyl-, sek.-Butyl-, tert.-Butyl-, Methoxy-, Ethoxy-, n-Propoxy-, Isopropoxy-, n-Butoxy-, Isobutoxy-, sek.-Butoxy-, tert.-Butoxygruppe oder ein Halogenatom, vorzugsweise ein Fluor- und/oder Chloratom, in 2- und/oder 4-Stellung substituiertem 1,3-Diaminobenzol oder in 2-Stellung substituiertem 1,4-Diaminobenzol, 1,5- und 1,8-Diaminonaphthalin, 4,4'-Diaminodiphenyl, 2,2'-, 2,4'- und 4,4'-Diaminodiphenylmethan und den entsprechenden Isomerengemischen und aromatischen Polyaminen, wie 1,3,5-Triaminobenzol, 2,4,6-Triaminotoluol und 1,3,5-Triaminonaphthalin.

Weiterhin im Fall der gegebenenfalls substituierten Alkylurethane die Reste von aliphatischen Monoaminen wie Methylamin, Ethylamin, Propylamin, Isopropylamin, n-Butylamin, sek.-Butylamin, tert.-

Butylamin, Isobutylamin, 2- und 3-Methylbutylamin, Neopentylamin, n-Pentylamin, 2-Methyl-pentylamin, sek.-iso-Amylamin, n-Hexylamin, 2-Methylhexylamin, 2-Ethylhexylamin, n-Heptylamin, n-Oktylamin, n-Nonylamin, n-Decylamin, n-Dodecylamin, 2-Phenylpropylamin, Benzylamin, Cyclopentylamin, Cyclohexylamin, tert.-Butylcyclohexylamin, von aliphatischen Diaminen wie Ethylendiamin, 1,3- und 1,2-Propylendiamin, 2,2-Dimethyl-1,3-propylendiamin, 1,4-Butylendiamin, 1,5-Pentamethylen-diamin, 1,6-Hexamethylen-diamin, 2,2,4-Trimethyl-1,6-hexamethylen-diamin, 1,8-Octamethylen-diamin, 1,10-Decylendiamin, 1,12-Dodecylen-diamin und 1,4-Hexahydroxylylen-diamin, cycloaliphatische Diamine, wie 1,2-, 1,3- und 1,4-Cyclohexan-diamin, 2,4- und 2,6-Hexahydrotoluylen-diamin sowie die entsprechenden Isomerengemische, aliphatisch-cycloaliphatische Diamine, wie 4,4'-, 2,4'- und 2,2'-Diamino-dicyclohexylmethan sowie die entsprechenden Isomerengemische, 2,2-Di-(4-aminocyclohexyl)-propan, 3-Aminomethyl-3,5,5-trimethylcyclohexylamin und Dicyclopentadienylverbindungen der Formel

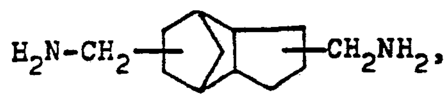

Heteroatome oder heterocyclische Reste gebunden enthaltende Diamine wie 3,3'-Diamino-dipropylether, gegebenenfalls substituierte N,N'-Bis-(aminoalkyl)-piperazin, z. B. N,N'-Bis-(2,2-dimethyl-3-aminopropyl)-piperazin und N,N'-Bis-(aminopropyl)-piperazin.

Als typische Beispiele für Arylurethane, die nach dem erfindungsgemäßen Verfahren thermisch spaltbar sind, seien genannt : N-Phenyl-methylurethan, N-Phenyl-ethylurethan, 3,5-Dichlorphenyl-ethylurethan, 4-Methylphenyl-ethylurethan, 2,4- und 2,6-Toluylen-di-methylurethan sowie die entsprechenden Isomerengemische, 2,4- und 2,6-Toluylen-di-ethylurethan, 2,4- und 2,6-Toluylen-di-butylurethan, 1,5-Naphthylen-di-ethylurethan, 4,4'-, 2,4'-, 2,2'-Methylendiphenyl-dimethylurethan, 4,4'-, 2,4'- und 2,2'-Methylen-diphenyl-diethylurethan, 4,4'-, 2,4'-, 2,2'-Methylen-diphenyl-dibutylurethan, 4,4'-, 2,4', 2,2'-Methylen-diphenyl-di-hexylurethan sowie die entsprechenden Isomerengemische.

Typische Beispiele für Alkylurethane sind : N-Methyl-octylurethan, N-Methyl-hexylurethan, N-Ethyldodecylurethan, N-Methyl-phenylurethan, N-Ethyl-phenylurethan, N-Propyl-phenylurethan, N-Propyl-decylurethan, N-Cyclohexyl-methylurethan, 1,6-Hexamethylen-dibutylurethan, 1,6-Hexamethylen-diethylurethan, 1,6-Hexamethylen-dimethylurethan, 2,2,4-Trimethyl-1,6-hexamethylen-dibutylurethan, 1,4-Hexahydroxylylen-diethylurethan, 1,4-Cyclohexyl-dimethylurethan, 1,4-Cyclohexyl-dibutylurethan und 3-(Butoxicarbonyl-aminomethyl)-3,5,5-trimethyl-cyclohexyl-butylurethan.

Nach dem erfindungsgemäßen Verfahren können Isocyanate mit hoher Reinheit in guten Ausbeuten wirtschaftlich vorteilhaft hergestellt werden.

Die hierbei erfindungsgemäß erhaltenen Monoisocyanate sind wertvolle Zwischenprodukte zur Herstellung von Schädlingsbekämpfungsmitteln, Farbstoffen und Hilfsmitteln ; die Diisocyanate werden vorzugsweise zur Herstellung von Polyurethan-Kunststoffen verwendet.

Die in den Beispielen genannten Teile beziehen sich auf das Gewicht. Gewichtsteile verhalten sich zu Volumenteilen wie Kilogramm zu Liter.

## Beispiel 1

Als Spaltapparatur wird eine Rührapparatur mit 500 Volumenteilen verwendet, in der 290 Teile Kohlenstoffperlen der Teilchengröße 0,5 bis 1,0 mm intensiv gerührt und auf 370 bis 375 °C erhitzt werden. Die Apparatur mündet über ein auf 310 bis 320 °C erhitztes Doppelmantelrohr in eine mit Drahtringen von 3 mm Durchmesser gefüllte Trennkolonne mit sieben Trennstufen, Verstärkungs- und Abtriebsteil, in der o-Xylol unter totalem Rückfluß kocht.

In das gerührte Kohlenstoffbett wird innerhalb 1 Stunde eine Lösung von 40 Teilen 2,4-Toluylendiethylurethan in 160 Teilen o-Xylol eingebracht, zusätzlich bläst man durch die Apparatur 100 Liter Stickstoff pro Liter Reaktionsgemisch und Stunde. Sobald die Temperatur am Kopf der Trennkolonne die Siedetemperatur des Alkohols erreicht hat, beginnt man mit der Entnahme. Nach beendeter Reaktion analysiert man die Isocyanatkonzentration in der Blase und das am Kopf entnommene Xylol/Alkohol-Gemisch mittels Gaschromatographie nach der Methode des « Internen Standard ». Es zeigt sich, daß 22,7 Teile 2,4-Toluylendiisocyanat (88,5 % der Theorie) und 13,3 Teile Ethanol (98,1 % der Theorie) gebildet wurden. Der Umsatz an 2,4-Toluylendiethylurethan betrug 98 % (ermittelt über Hochdruckflüssigchromatographie).

## Beispiele 2 bis 8

Man verfährt wie in Beispiel 1, wobei jedoch andere Urethane und Lösungs- bzw. Strippmittel zum Einsatz kommen. Die Ergebnisse sind in der Tabelle 1 aufgeführt.

Tabelle 1

| Beispiel Nr. | Urethan | Lösungs- bzw. Strippmittel | Umsatz % | Isocyanat | Ausbeute % |
|---|---|---|---|---|---|
| 2 | N-Phenylmethylurethan | Toluol | 76.3 | Phenylisocyanat | 99.1 |
| 3 | 2,4-Bis-(butoxycarbonyl-amino)-toluol | o-Xylol | 94.1 | 2.4-Toluylen-diisocyanat | 92.2 |
| 4 | N-(3,5-Dichlorphenyl)-methylurethan | Ethylenglykol-dimethylether | 89.3 | 3,5-Dichlorphenyl-isocyanat | 96.4 |
| 5 | Hexamethylen-1,6-diethyl-urethan | Toluol | 67.2 | Hexamethylendiiso-cyanat | 71.5 |
| 6 | Hexamethylen-1,6-dibutyl-urethan | o-Xylol | 81.0 | Hexamethylendiiso-cyanat | 89.8 |
| 7 | N-Propylphenylurethan | Toluol | 76.7 | Propylisocyanat | 98.7 |
| 8 | 1-(Butoxicarbonylamino)-3-(Butoxicarbonylaminomethyl)-3,5,5-trimethylcyclohexan | o-Xylol | 58.8 | Isophorondiisocyanat | 71.6 |

Beispiel 9

In einen von außen elektrisch auf die Innentemperatur von 350 °C beheizten, zylinderförmigen Wirbelbehälter mit einem Durchmesser von 6 cm und einer Höhe von 30 cm, ausgestattet mit einer Fritte als Anströmboden und einer Düse, die seitlich 5 cm über dem Anströmboden angeordnet ist, werden 200 g Kohlenstoffstaub mit einem durchschnittlichen Korndurchmesser von 0,1 bis 0,5 mm als Vorlage eingebracht. Das in einem Wärmetauscher vorerhitzte Wirbelgas Stickstoff wird über den Anströmboden zugeführt. Man düst während einer Stunde 400 Gew.-Teile einer Lösung aus 10 Gew. % Toluylen-2,4-dibutylurethan in Xylol ein und kondensiert — nach Entstaubung mit Hilfe eines Drahtgewebefilters — das Gemisch aus entstandenem Toluylen-2,4-diisocyanat, Butanol, Xylol und carbamatgruppenhaltigem Monoisocyanat fraktioniert in vier Vorlagen. Die Fraktionen werden gaschromatographisch nach der Methode des « Internen Standard » analysiert, wobei sich zeigt, daß 19,1 Teile Toluylen-2,4-diisocyanat (88,4 % d. Th.), 16,4 Teile Butanol (89,4 % d. Th.) sowie carbamathaltiges Monoisocyanat gebildet wird.

Beispiele 10 bis 12

Man verfährt wie in Beispiel 9, wobei jedoch andere Urethane, Lösungs- und Strippmittel und Spalttemperaturen zum Einsatz kommen. Die Ergebnisse sind in Tabelle 2 aufgeführt.

Tabelle 2

| Bsp. Nr. | Urethane | Lösungsmittel | Spalttemp. °C | Isocyanate | Ausbeute % |
|---|---|---|---|---|---|
| 10 | Toluylen-2,4-diethylurethan | Toluol | 370 | 2,4-Toluylendiisocyanat | 91,7 |
| 11 | N-Phenylmethylurethan | Toluol | 320 | Phenylisocyanat | 98,7 |
| 12 | Hexamethylen-1,6-dibutylurethan | Ethylenglykoldimethylether | 380 | 1,6-Hexamethylendiisocyanat | 83,8 |

**Patentansprüche**

1. Verfahren zur Herstellung von Isocyanaten durch thermische Spaltung von Urethanen bei Temperaturen von 175 bis 600 °C, dadurch gekennzeichnet, daß man die Spaltung in Gegenwart von Kohlenstoff durchführt.

2. Verfahren nach Anspruch 1, dadurch gekennzeichnet, daß der Kohlenstoff pulverförmig ist und eine durchschnittliche Korngröße von 0,01 bis 10 mm aufweist.

3. Verfahren nach Anspruch 1, dadurch gekennzeichnet, daß die Spaltung in einem gerührten Kohlebett erfolgt.

4. Verfahren nach Anspruch 1, dadurch gekennzeichnet, daß der Kohlenstoff als Feststoffschüttung in einem Wirbelbett vorliegt.

5. Verfahren nach Anspruch 1, dadurch gekennzeichnet, daß die Spaltung in einer Wirbelschicht erfolgt.

6. Verfahren nach Anspruch 1, dadurch gekennzeichnet, daß man die Spaltprodukte Isocyanat und Alkohol mit Hilfe eines Strippmittels aus dem Reaktionsgemisch abtrennt.

7. Verfahren nach Anspruch 6, dadurch gekennzeichnet, daß das Strippmittel einen Siedepunkt besitzt, der zwischen den Siedepunkten des bei der Spaltung entstehenden Isocyanates und Alkohols liegt.

8. Verfahren nach Anspruch 6, dadurch gekennzeichnet, daß man als Strippmittel ein unter den Reaktionsbedingungen inertes Lösungsmittel verwendet.

9. Verfahren nach Anspruch 6, dadurch gekennzeichnet, daß man zur Abtrennung der Spaltprodukte zusätzlich zum Strippmittel ein Inertgas verwendet.

10. Verfahren nach Anspruch 1, dadurch gekennzeichnet, daß man als Urethane Alkyl-mono- und/oder -polyurethane oder Aryl-mono- und/oder -polyurethane spaltet.

**Claims**

1. A process for the preparation of an isocyanate by thermally cleaving a urethane at temperatures of from 175 to 600 °C, wherein the thermal cleavage is carried out in the presence of carbon.

2. A process as claimed in claim 1, wherein the carbon is in the form of a powder having an average grain size of from 0.01 to 10 mm.

3. A process as claimed in claim 1, wherein cleavage is carried out in an agitated carbon bed.

4. A process as claimed in claim 1, wherein the carbon is present as bulk material in a fluidized bed.

5. A process as claimed in claim 1, wherein cleavage is carried out in a fluidized bed.

6. A process as claimed in claim 1, wherein the cleavage products, the isocyanate and alcohol, are separated from the reaction mixture with the aid of a stripping agent.

7. A process as claimed in claim 6, wherein the stripping agent has a boiling point which lies between the boiling points of the isocyanate and the alcohol formed in the cleavage.

8. A process as claimed in claim 6, wherein a solvent which is inert under the reaction conditions is used as the stripping agent.

9. A process as claimed in claim 6, wherein an inert gas is used in addition to the stripping agent in order to separate the cleavage products.

10. A process as claimed in claim 1, wherein the urethanes cleaved are alkyl monourethanes and/or polyurethanes or aryl monourethanes and/or polyurethanes.

**Revendications**

1. Procédé de préparation d'isocyanates par scission thermique d'uréthannes, à des températures de 175 à 600 °C, caractérisé par le fait que l'on opère la scission en présence de carbone.

2. Procédé selon la revendication 1, caractérisé par le fait que le carbone est pulvérulent et présente une grosseur de grain moyenne de 0,01 à 10 mm.

3. Procédé selon la revendication 1, caractérisé par le fait que la scission se produit dans un lit de carbone agité.

4. Procédé selon la revendication 1, caractérisé par le fait que le carbone se présente sous forme d'une masse en vrac solide dans un lit en mouvement turbulent.

5. Procédé selon la revendication 1, caractérisé par le fait que la scission s'effectue dans une couche en mouvement turbulent.

6. Procédé selon la revendication 1, caractérisé par le fait que l'on sépare les produits de scission, isocyanate et alcool, du mélange de réaction, au moyen d'un agent de fractionnement.

7. Procédé selon la revendication 6, caractérisé par le fait que l'agent de fractionnement possède un point d'ébullition compris entre les points d'ébullition de l'isocyanate et de l'alcool résultant de la scission.

8. Procédé selon la revendication 6, caractérisé par le fait que l'on utilise, comme agent de fractionnement, un solvant inerte dans les conditions de réaction.

9. Procédé selon la revendication 6, caractérisé par le fait que, pour la séparation des produits de scission, on utilise un gaz inerte en plus de l'agent de fractionnement.

10. Procédé selon la revendication 1, caractérisé par le fait que l'on scinde, comme uréthanne, de l'alkyl-mono- et/ou -polyuréthanne ou aryl-mono- et/ou -polyuréthanne.